(19) Europäisches Patentamt
European Patent Office
Office européen des brevets

(11) EP 2 903 959 B1

(12) **EUROPÄISCHE PATENTSCHRIFT**

(45) Veröffentlichungstag und Bekanntmachung des Hinweises auf die Patenterteilung:
**23.08.2017 Patentblatt 2017/34**

(21) Anmeldenummer: **13773238.4**

(22) Anmeldetag: **04.10.2013**

(51) Int Cl.:
***C07C 51/36*** (2006.01) ***C07C 61/08*** (2006.01)
***C07C 67/303*** (2006.01) ***C07C 69/75*** (2006.01)

(86) Internationale Anmeldenummer:
**PCT/EP2013/070667**

(87) Internationale Veröffentlichungsnummer:
**WO 2014/053618 (10.04.2014 Gazette 2014/15)**

(54) **VERFAHREN ZUR HERSTELLUNG VON CYCLOHEXANPOLYCARBONSÄURE-DERIVATEN MIT GERINGEM NEBENPRODUKTANTEIL**

METHOD FOR THE PREPARATION OF CYCLOHEXANE POLYCARBOXYLIC ACID DERIVATIVES WITH LOW PROPORTION OF BY-PRODUCT

PROCÉDÉ DE FABRICATION DE DÉRIVÉS POLYCARBOXYLÉS DU CYCLOHEXANE MENANT A DES PRODUITS SECONDAIRES EN FAIBLES QUANTITÉS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **05.10.2012 EP 12187399**

(43) Veröffentlichungstag der Anmeldung:
**12.08.2015 Patentblatt 2015/33**

(73) Patentinhaber: **BASF SE**
**67056 Ludwigshafen am Rhein (DE)**

(72) Erfinder:
- **BOCK, Martin**
**67063 Ludwigshafen (DE)**
- **BREITSCHEIDEL, Boris**
**67165 Waldsee (DE)**

(74) Vertreter: **BASF IP Association**
**BASF SE**
**G-FLP-C006**
**67056 Ludwigshafen (DE)**

(56) Entgegenhaltungen:
**EP-A1- 1 323 700** **EP-A1- 2 316 811**
**WO-A1-94/29261** **WO-A2-2011/082991**
**US-A1- 2007 255 070** **US-A1- 2008 188 601**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 903 959 B1

## Beschreibung

Die vorliegende Erfindung betrifft ein kontinuierliches Verfahren zur Herstellung von wenigstens einer Cyclohexanpolycarbonsäure oder einem Derivat davon durch Inkontaktbringen wenigstens einer entsprechenden Benzolpolycarbonsäure oder eines Derivates davon mit einem Wasserstoff enthaltenden Gas in Gegenwart wenigstens eines Schalenkatalysators, enthaltend ein Aktivmetall ausgewählt aus der Gruppe bestehend aus Ruthenium, Rhodium, Palladium, Platin und Mischungen davon, aufgebracht auf ein Trägermaterial enthaltend Siliziumdioxid, wobei das Porenvolumen des Trägermaterials 0,6 bis 1,0 ml/g, bestimmt durch Hg-Porosimetrie, beträgt, die BET-Oberfläche 280 bis 500 $m^2$/g beträgt, mindestens 90% der vorhandenen Poren einen Durchmesser von 6 bis 12 nm aufweisen, und 40 bis 70 Gew.-% des Aktivmetalls, bezogen auf die Gesamtmenge des Aktivmetalls, in der Schale des Katalysators bis zu einer Eindringtiefe von 200 $\mu$m vorliegen, wobei das Inkontaktbringen bei einer Leerrohrgeschwindigkeit von 20 bis 50 m/h erfolgt, wobei es sich bei dem wenigstens einen Derivat der Benzolpolycarbonsäure um Mono-, Di-, Tri-, Tetraester und Anhydride der Benzolpolycarbonsäure handelt.

Ferner betrifft die vorliegende Erfindung auch ausgewählte Vertreter der erhaltenen Cyclohexanpolycarbonsäuren oder einem Derivat davon, sowie die Verwendung der erhaltenen Cyclohexanpolycarbonsäuren oder einem Derivat davon als Weichmacher in Kunststoffen.

In der US 5,286,898 und der US 5,319,129 wird Dimethylterephthalat an geträgerten Pd-Katalysatoren, die mit Ni, Pt und/oder Ru versetzt sind, bei Temperaturen > 140 °C und einem Druck zwischen 50 und 170 bar zum entsprechenden Hexahydrodimethylterephthalat hydriert. In der DE-A 28 23 165 werden aromatische Carbonsäureester an geträgerten Ni-, Ru-, Rh-, und/ oder Pd-Katalysatoren zu den entsprechenden cycloaliphatischen Carbonsäureestern bei 70 bis 250 °C und 30 bis 200 bar hydriert. In der US 3,027,398 wird die Hydrierung von Dimethylterephthalat an geträgerten Ru-Katalysatoren bei 110 bis 140 °C und 35 bis 105 bar beschrieben.

Die EP-A 0 603 825 betrifft ein Verfahren zur Herstellung von 1,4-Cyclohexandicarbonsäure durch Hydrierung von Terephthalsäure unter Verwendung eines geträgerten PalladiumKatalysators, wobei als Träger Aluminiumoxid, Siliziumdioxid oder Aktivkohle verwendet wird. Das dort beschriebene Verfahren ist insbesondere dadurch charakterisiert, dass die in einer ersten Stufe erhaltene 1,4-Cyclohexandicarbonsäure enthaltende Lösung mit Dampf in Kontakt gebracht wird und dadurch in dieser Lösung enthaltene Verunreinigungen extrahiert werden. Dieses Verfahren ist jedoch nur auf Säuren anwendbar, da bei der Anwendung auf Derivate, wie z. B. Ester, Anhydride, usw. die Gefahr von Hydrolyse besteht. In der EP 1 042 273 ist ein Verfahren zur Hydrierung von Polycarbonsäure-Derivaten unter Verwendung eines Makroporen aufweisenden Katalysators beschrieben. Das Verfahren zeichnet sich durch eine hohe Raum-ZeitAusbeute und eine hohe Selektivität aus.

Einige Cyclohexanpolycarbonsäure-Derivate sowie deren Verwendung als Weichmacher sind ebenfalls aus dem Stand der Technik bekannt. So sind Cyclohexandicarbonsäuredimethyl- oder diethylester (DE-A 28 23 165), Cyclohexan-1,2-dicarbonsäuredi(2-ethylhexyl)ester (DE-A 12 63 296) und Cyclohexan-1,2-dicarbonsäurediisononylester (EP 1 042 273) und deren Verwendung als Weichmacher in Kunstoffen beschrieben.

US 2008/0188601 A1 offenbart eine Mischung von Diisononylestern von 1,2-Cyclohexandicarbonsäure, ein Verfahren zu deren Herstellung sowie die Verwendungen der Mischungen. Die Hydrierung wird in der flüssigen Phase oder in der flüssig-gasförmigen Phase durchgeführt. Um eine einheitliche Verteilung der Reaktionspartner zu gewährleisten, wird die Reaktion bei einer hohen Oberflächengeschwindigkeit von 15 bis 120$m^3$, bevorzugt 20 bis 80 $m^3$, pro $m^2$ Querschnitt des leeren Reaktors und Stunde durchgeführt.

WO 2011/082991 A2 betrifft einen Schalenkatalysator, enthaltend ein Aktivmetall ausgewählt aus der Gruppe bestehend aus Ruthenium, Rhodium, Palladium, Platin und Mischungen davon, aufgebracht auf ein Trägermaterial enthaltend Siliziumdioxid, wobei das Porenvolumen des Trägermaterials 0,6 bis 1,0 ml/g, bestimmt durch Hg-Porosimetrie, beträgt, die BET-Oberfläche 280 bis 500 $m^2$/g beträgt, und mindestens 90 % der vorhandenen Poren einen Durchmesser von 6 bis 12 nm aufweisen sowie ein Verfahren zur Hydrierung einer organischen Verbindung, die mindestens eine hydrierbare Gruppe enthält unter Verwendung dieses Schalenkatalysators.

Der Nachteil der oben beschriebenen Herstellverfahren bzw. der mit Hilfe dieser Verfahren hergestellten Cyclohexanpolycarbonsäure-Derivate besteht in einem hohen Anteil von Nebenprodukten im Endprodukt, insbesondere einem hohen Anteil an Hexahydrophthalid und Isononylalkohol, die ggf. eine aufwendige Nachreinigung erfordert. Bedingt durch diesen hohen Anteil an Nebenprodukten weisen die durch Verfahren gemäß Stand der Technik hergestellten Cyclohexanpolycarbonsäure-Derivate bei der Verwendung als Weichmacher nachteilige anwendungstechnische Eigenschaften auf, wie eine hohe Flüchtigkeit und eine schlechte Verträglichkeit mit Kunststoffen, beispielweise PVC. Dadurch sind die aus dem Stand der Technik bekannten Cyclohexanpolycarbonsäure-Derivate weniger gut für sensible Anwendungen in Kontakt mit Menschen geeignet, z. B. für Kinderspielzeug, Lebensmittelverpackungen oder medizinische Artikel.

Der vorliegenden Erfindung lag die Aufgabe zugrunde, ein Verfahren zur Hydrierung von Benzolpolycarbonsäuren oder Derivaten davon zur Verfügung zu stellen, mit dem die entsprechenden Cyclohexanpolycarbonsäuren oder Derivate davon in hoher Reinheit, d. h. mit einem geringen Anteil an Nebenkomponenten, insbesondere mit einem

niedrigen Anteil an Hexahydrophthalid und Isononylalkohol, erhalten werden können, damit sich diese insbesondere zur Verwendung als Weichmacher in Kunststoffen eignen, insbesondere für sensible Anwendungen im Kontakt mit Menschen.

Es wurde gefunden, dass man Cyclohexanpolycarbonsäuren oder Derivate davon mit einem verringerten Anteil an Nebenprodukten, insbesondere Hexahydrophthalid und Isononanol, erhält, wenn die Hydrierung der entsprechenden Benzolpolycarbonsäuren oder deren Derivate, d. h. das Inkontaktbringen dieser Verbindungen mit einem Wasserstoff enthaltenden Gas, in Gegenwart eines speziellen Schalenkatalysators und bei einer Leerrohrgeschwindigkeit von 20 bis 50 m/h erfolgt.

Demgemäß betrifft die vorliegende Erfindung ein kontinuierliches Verfahren zur Herstellung von wenigstens einer Cyclohexanpolycarbonsäure oder einem Derivat davon durch Inkontaktbringen wenigstens einer entsprechenden Benzolpolycarbonsäure oder eines Derivates davon mit einem Wasserstoff enthaltenden Gas in Gegenwart wenigstens eines Schalenkatalysators, enthaltend ein Aktivmetall ausgewählt aus der Gruppe bestehend aus Ruthenium, Rhodium, Palladium, Platin und Mischungen davon, aufgebracht auf ein Trägermaterial enthaltend Siliziumdioxid, wobei das Porenvolumen des Trägermaterials 0,6 bis 1,0 ml/g, bestimmt durch Hg-Porosimetrie, beträgt, die BET-Oberfläche 280 bis 500 $m^2/g$ beträgt, mindestens 90% der vorhandenen Poren einen Durchmesser von 6 bis 12 nm aufweisen, und 40 bis 70 Gew.-% des Aktivmetalls, bezogen auf die Gesamtmenge des Aktivmetalls, in der Schale des Katalysators bis zu einer Eindringtiefe von 200 $\mu$m vorliegen, wobei das Inkontaktbringen bei einer Leerrohrgeschwindigkeit von 20 bis 50 m/h erfolgt, wobei es sich bei dem wenigstens einen Derivat der Benzolpolycarbonsäure um Mono-, Di-, Tri-, Tetraester und Anhydride der Benzolpolycarbonsäure handelt.

Das erfindungsgemäße Inkontaktbringen der wenigstens einen Benzopolycarbonsäure oder eines Derivates davon mit einem Wasserstoff enthaltenden Gas resultiert in einer Hydrierung dieser Verbindungen, um die gewünschte wenigstens eine Cyclohexanpolycarbonsäure oder ein Derivat davon zu erhalten. Erfindungsgemäß bevorzugt wird nur das aromatische System hydriert, d. h. reduziert, um das entsprechende gesättigte cycloaliphatische System zu erhalten, d. h. ggf. weitere in dem wenigstens einen Substrat vorliegende reduzierbare Gruppen werden erfindungsgemäß bevorzugt nicht reduziert.

In dem erfindungsgemäßen Verfahren zur Herstellung von wenigstens einer Cyclohexanpolycarbonsäure oder einem Derivat davon durch Inkontaktbringen wenigstens einer entsprechenden Benzolpolycarbonsäure oder eines Derivates davon mit einem Wasserstoff enthaltenden Gas wird wenigstens ein Schalenkatalysator, enthaltend ein Aktivmetall ausgewählt aus der Gruppe bestehend aus Ruthenium, Rhodium, Palladium, Platin und Mischungen davon, aufgebracht auf ein Trägermaterial enthaltend Siliziumdioxid, wobei das Porenvolumen des Trägermaterials 0,6 bis 1,0 ml/g, bestimmt durch Hg-Porosimetrie, beträgt, die BET-Oberfläche 280 bis 500 $m^2/g$ beträgt, mindestens 90% der vorhandenen Poren einen Durchmesser von 6 bis 12 nm aufweisen, und 40 bis 70 Gew.-% des Aktivmetalls, bezogen auf die Gesamtmenge des Aktivmetalls, in der Schale des Katalysators bis zu einer Eindringtiefe von 200 $\mu$m vorliegen, eingesetzt.

Erfindungsgemäß geeignete Schalenkatalysatoren mit einem Aktivmetall auf einem Träger sind in der WO2011/082991 genannt. Der Inhalt der WO2011/082991 wird durch Bezugnahme vollständig in die vorliegende Anmeldung einbezogen.

Der erfindungsgemäß eingesetzte Schalenkatalysator enthält ein Aktivmetall ausgewählt aus der Gruppe bestehend aus Ruthenium, Rhodium, Palladium, Platin und Mischungen davon, bevorzugt Ruthenium, ganz besonders bevorzugt Ruthenium als einziges Aktivmetall.

In dem erfindungsgemäß eingesetzten Schalenkatalysator beträgt die Menge des Aktivmetalls im Allgemeinen weniger als 1 Gew.-%, bevorzugt 0,1 bis 0,5 Gew.-%, besonders bevorzugt 0,25 bis 0,35 Gew.-%, bezogen auf das Gesamtgewicht des Katalysators.

Schalenkatalysatoren sind dem Fachmann an sich bekannt. Im Rahmen der vorliegenden Erfindung bedeutet der Begriff "Schalenkatalysator", dass das vorhandene mindestens eine Aktivmetall, bevorzugt Ruthenium, zum überwiegenden Teil in einer äußeren Schale des Trägermaterials vorliegt.

In dem erfindungsgemäß eingesetzten Schalenkatalysatoren liegen 40 bis 70 Gew.-%, bevorzugt 50 bis 60 Gew.-% des Aktivmetalls, bezogen auf die Gesamtmenge des Aktivmetalls, in der Schale des Katalysators bis zu einer Eindringtiefe von 200 $\mu$m vor. In einer besonders bevorzugten Ausführungsform liegen 60 bis 90 Gew.- %, ganz besonders bevorzugt 70 bis 80 Gew.-% des Aktivmetalls, bezogen auf die Gesamtmenge des Aktivmetalls, in der Schale des Katalysators bis zu einer Eindringtiefe von 500 $\mu$m vor. Die vorstehend genannten Daten werden mittels SEM (scanning electron microscopy) EPMA (electron probe microanalysis) - EDXS (energy dispersive X-ray spectroscopy) ermittelt und stellen gemittelte Werte dar. Weitere Informationen bezüglich den vorstehend genannten Messverfahren und Techniken sind zum Beispiel in Spectroscopy in Catalysis von J.W. Niemantsverdriet, VCH, 1995 oder Handbook of Microscopy von S. Amelinckx et al. offenbart. Zur Ermittlung der Eindringtiefe der Aktivmetallteilchen werden mehrere Katalysatorteilchen (z. B. 3, 4 oder 6) angeschliffen. Mittels Linescans werden dann die Profile der Aktivmetall/Si Konzentrationsverhältnisse erfasst. Auf jeder Messlinie werden mehrere, zum Beispiel 15 bis 20, Messpunkte in gleichen Abständen gemessen, die Messfleckgröße beträgt circa 10 $\mu$m * 10 $\mu$m. Nach Integration der Aktivmetallmenge über die

Tiefe kann die Häufigkeit des Aktivmetalls in einer Zone bestimmt werden.

Ganz besonders bevorzugt beträgt die Menge des Aktivmetalls, bezogen auf das Konzentrationsverhältnis von Aktivmetall zu Si, an der Oberfläche des Schalenkatalysators, 2 bis 25%, bevorzugt 4 bis 10%, besonders bevorzugt 4 bis 6%, ermittelt mittels SEM EPMA - EDXS. Die Oberflächenanalyse erfolgt mittels Bereichsanalysen von Bereichen von 800 $\mu$m * 2000 $\mu$m und mit einer Informationstiefe von circa 2 $\mu$m. Die Elementzusammensetzung wird in Gew.-% (normiert auf 100 Gew.-%) bestimmt. Das mittlere Konzentrationsverhältnis (Aktivmetall/Si) wird über 10 Messbereiche gemittelt.

Unter der Oberfläche des Schalenkatalysators ist im Sinne der vorliegenden Erfindung die äußere Schale des Katalysators bis zu einer Eindringtiefe von circa 2 $\mu$m zu verstehen. Diese Eindringtiefe entspricht der Informationstiefe bei der vorstehend erwähnten Oberflächenanalyse.

Ganz besonders bevorzugt ist ein Schalenkatalysator, worin die Menge des Aktivmetalls, bezogen auf das Gewichtsverhältnis von Aktivmetall zu Si (Gew./Gew. in%), an der Oberfläche des Schalenkatalysators 4 bis 6% beträgt, in einer Eindringtiefe von 50 $\mu$m 1,5 bis 3% und im Bereich von 50 bis 150 $\mu$m Eindringtiefe 0,5 bis 2%, ermittelt mittels SEM EPMA (EDXS), beträgt. Die genannten Werte stellen gemittelte Werte dar.

Des Weiteren nimmt die Größe der Aktivmetallteilchen bevorzugt mit zunehmender Eindringtiefe ab, ermittelt mittels (FEG)-TEM-Analyse.

Das Aktivmetall liegt in dem erfindungsgemäßen Schalenkatalysator bevorzugt teilweise oder vollständig kristallin vor. In bevorzugten Fällen kann in der Schale des erfindungsgemäßen Schalenkatalysators mittels SAD (Selected Area Diffraction) feinstkristallines Aktivmetall nachgewiesen werden.

Der erfindungsgemäß bevorzugte Schalenkatalysator kann zusätzlich Erdalkalimetallionen ($M^{2+}$), also M = Be, Mg, Ca, Sr und/oder Ba, insbesondere Mg und/oder Ca, ganz besonders Mg enthalten. Der Gehalt an Erdalkalimetallion/en ($M^{2+}$) im Katalysator beträgt bevorzugt 0,01 bis 1 Gew.-%, insbesondere 0,05 bis 0,5 Gew.-%, ganz besonders 0,1 bis 0,25 Gew.-%, jeweils bezogen auf das Gewicht des Siliziumdioxid-Trägermaterials.

Ein wesentlicher Bestandteil der erfindungsgemäß eingesetzten Schalenkatalysatoren ist das Trägermaterial enthaltend Siliziumdioxid, bevorzugt amorphes Siliziumdioxid. Unter dem Begriff "amorph" versteht man in diesem Zusammenhang, dass der Anteil kristalliner Siliziumdioxid-Phasen weniger als 10 Gew.-% des Trägermaterials ausmacht. Die zur Herstellung der Katalysatoren verwendeten Trägermaterialien können allerdings Überstrukturen aufweisen, die durch regelmäßige Anordnung von Poren im Trägermaterial gebildet werden.

Als Trägermaterialien kommen grundsätzlich amorphe Siliziumdioxid-Typen in Betracht, die mindestens zu 90 Gew.-% aus Siliziumdioxid bestehen, wobei die verbleibenden höchstens 10 Gew.-%, vorzugsweise nicht mehr als 5 Gew.-%, des Trägermaterials auch ein anderes oxidisches Material sein können, z. B. MgO, CaO, $TiO_2$, $ZrO_2$ und/oder $Al_2O_3$.

In einer bevorzugten Ausführungsform der Erfindung ist das Trägermaterial halogenfrei, insbesondere chlorfrei, d. h. der Gehalt an Halogen im Trägermaterial beträgt im Allgemeinen weniger als 500 Gew.-ppm, z. B. im Bereich von 0 bis 400 Gew.-ppm. Somit ist ein Schalenkatalysator bevorzugt, der weniger als 0,05 Gew.-% Halogenid (ionenchromatographisch bestimmt), bezogen auf das Gesamtgewicht des Katalysators, enthält. Besonders bevorzugt liegt der Halogenidgehalt des Trägermaterials unterhalb der analytischen Nachweisgrenze. Bevorzugt sind Trägermaterialien enthaltend Siliziumdioxid, die eine spezifische Oberfläche im Bereich von 280 bis 500 $m^2$/g, besonders bevorzugt 280 bis 400 $m^2$/g, ganz besonders bevorzugt 300 bis 350 $m^2$/g, (BET-Oberfläche nach DIN 66131) aufweisen. Sie können sowohl natürlichen Ursprungs als auch künstlich hergestellt worden sein. Beispiele für geeignete amorphe Trägermaterialien auf Basis von Siliziumdioxid sind Kieselgele, Kieselgur, pyrogene Kieselsäuren und Fällungskieselsäuren. In einer bevorzugten Ausführungsform der Erfindung weisen die Katalysatoren Kieselgele als Trägermaterialien auf.

Das Porenvolumen des Trägermaterials beträgt erfindungsgemäß 0,6 bis 1,0 ml/g, bevorzugt 0,65 bis 0,9 ml/g, beispielsweise 0,7 bis 0,8 ml/g, bestimmt durch Hg-Porosimetrie (DIN 66133). In dem erfindungsgemäß bevorzugten Schalenkatalysator weisen mindestens 90% der vorhandenen Poren einen Porendurchmesser von 6 bis 12 nm, bevorzugt 7 bis 11 nm, besonders bevorzugt 8 bis 10 nm, auf. Der Porendurchmesser kann nach dem Fachmann bekannten Verfahren bestimmt werden, beispielsweise durch Hg-Porosimetrie gemäß DIN 66133 oder $N_2$-Physisorption gemäß DIN 66131. In einer bevorzugten Ausführungsform weisen mindestens 95%, besonders bevorzugt mindestens 98%, der vorhandenen Poren einen Porendurchmesser von 6 bis 12 nm, bevorzugt 7 bis 11 nm, besonders bevorzugt 8 bis 10 nm, auf.

In dem erfindungsgemäß bevorzugten Schalenkatalysator liegen in einer bevorzugten Ausführungsform keine Poren vor, die kleiner als 5 nm sind. Des Weiteren liegen in dem erfindungsgemäß bevorzugten Schalenkatalysator keine Poren vor, die grösser als 25 nm, insbesondere grösser als 15 nm, sind. In diesem Zusammenhang bedeutet "keine Poren", dass mit üblichen Messverfahren, beispielsweise Hg-Porosimetrie gemäß DIN 66133 oder $N_2$-Physisorption gemäß DIN 66131 keine Poren mit diesen Durchmessern gefunden werden. In dem erfindungsgemäß eingesetzten Schalenkatalysator liegen im Rahmen der Messgenauigkeit der verwendeten Analytik bevorzugt keine Makroporen, sondern ausschließlich Mesoporen vor.

Bei dem erfindungsgemäß bevorzugten Schalenkatalysators werden besonders bevorzugt Formkörper aus

dem Trägermaterial, die z. B. durch Extrudieren, Strangpressen oder Tablettieren erhältlich sind und die z. B. die Form von Kugeln, Tabletten, Zylindern, Strängen, Ringen bzw. Hohlzylindern, Sternen und dergleichen, besonders bevorzugt Kugeln, aufweisen können, eingesetzt. Die Abmessungen dieser Formkörper bewegen sich üblicherweise im Bereich von 0,5 mm bis 25 mm. Bevorzugt werden Katalysatorkugeln mit Kugeldurchmessern von 1,0 bis 6,0 mm, besonders bevorzugt 2,5 bis 5,5 mm, eingesetzt. In dem erfindungsgemäß bevorzugten Schalenkatalysator beträgt Dispersität des Aktivmetalls bevorzugt 30 bis 60%, besonders bevorzugt 30 bis 50%. Verfahren zur Messung der Dispersität des Aktivmetalls sind dem Fachmann an sich bekannt, beispielsweise durch Puls-Chemisorption, wobei die Bestimmung der Edelmetalldispersion (spezifische Metalloberfläche, Kristallitgrösse) mit CO Pulsmethode durchgeführt wird (DIN 66136(1-3)).

In dem erfindungsgemäß bevorzugten Schalenkatalysator beträgt die Oberfläche des Aktivmetalls bevorzugt 0,2 bis 0,8 $m^2/g$, besonders bevorzugt 0,3 bis 0,7 $m^2/g$. Verfahren zur Messung der Oberfläche des Aktivmetalls sind dem Fachmann an sich bekannt.

Die Herstellung der erfindungsgemäß bevorzugten Schalenkatalysatoren erfolgt beispielsweise dadurch, dass man zunächst das Trägermaterial mit einer Lösung enthaltend eine Vorläuferverbindung des Aktivmetalls ein- oder mehrfach tränkt, den erhaltenen Feststoff trocknet und anschließend reduziert. Die einzelnen Verfahrensschritte sind dem Fachmann an sich bekannt und in der WO 2011/082991 beschrieben.

Die Verfahrensführung:

Für das erfindungsgemäße Verfahren ist es erfindungswesentlich, dass das Inkontaktbringen der wenigstens einen Benzolpolycarbonsäure oder eines Derivates davon mit dem Wasserstoff enthaltenden Gas, d. h. die Hydrierung der wenigstens einen Benzolpolycarbonsäure oder eines Derivates davon, bei einer Leerohrgeschwindigkeit von 20 bis 50 m/h im ersten Reaktor erfolgt, bevorzugt bei einer Leerrohrgeschwindigkeit von 20 bis 45 m/h, besonders bevorzugt wird das erfindungsgemäße Verfahren bei einer Leerrohrgeschwindigkeit von 20 bis 40 m/h, durchgeführt.

Die Leerrohrgeschwindigkeit ist erfindungsgemäß wie folgt definiert:

$$\text{Leerrohrgeschwindigkeit} = V(\text{Zulauf})/(A(1.\ \text{Reaktor})*t),$$

wobei V(Zulauf 1. Reaktor) die Summe des Volumens des Eduktes, d. h. der wenigstens einen Benzolpolycarbonsäure oder eines Derivates davon, und des Lösungsmittels, d. h. des nach Durchgang des 1. Reaktors zurückgeführten Produktes, jeweils in $m^3$, ist, A(Reaktor) die Querschnittsfläche des Reaktors in $m^2$, und t die Zeit in Stunden, in der V(Zulauf) den Querschnitt A passiert, bedeuten. Die Leerrohrgeschwindigkeit ist daher ein Maß für die Strömungsgeschwindigkeit im Reaktor.

Im Rahmen des erfindungsgemäßen Verfahrens wird das Inkontaktbringen, d. h. die Hydrierung, im Allgemeinen bei einer Temperatur von 50 bis 250 °C, vorzugsweise 70 bis 180 °C durchgeführt. Die dabei verwendeten Drücke liegen in der Regel bei oberhalb von 10 bar, vorzugsweise zwischen 20 bis 80 bar, besonders bevorzugt zwischen 30 und 50 bar.

Die vorliegende Erfindung betrifft daher bevorzugt das erfindungsgemäße Verfahren, wobei das Inkontaktbringen, d. h. die Hydrierung, bei einer Temperatur von 50 bis 250 °C, vorzugsweise 70 bis 180 °C und einem Druck oberhalb von 10 bar, vorzugsweise zwischen 20 bis 80 bar, besonders bevorzugt zwischen 30 und 50 bar, durchgeführt wird.

Das erfindungsgemäße Verfahren wird kontinuierlich durchgeführt.

Vorzugsweise führt man das erfindungsgemäße Verfahren in Rieselreaktoren oder in gefluteter Fahrweise nach der Festbettfahrweise durch. Das Wasserstoff enthaltende Gas kann dabei sowohl im Gleichstrom mit der Lösung des zu hydrierenden Edukts als auch im Gegenstrom über den Katalysator geleitet werden.

Bei der kontinuierlichen Verfahrensführung beträgt die Menge der wenigstens einen zur Hydrierung vorgesehenen Benzolpolycarbonsäure oder einem Derivat davon vorzugsweise 0,05 bis 3 kg pro Liter Katalysator pro Stunde, weiter bevorzugt 0,1 bis 1 kg pro Liter Katalysator pro Stunde.

Als Hydriergase können beliebige Wasserstoff in freier Form enthaltende Gase verwendet werden, die keine schädlichen Mengen an Katalysatorgiften, wie beispielsweise CO, aufweisen. Bei spielsweise können Reformerabgase verwendet werden. Vorzugsweise wird reiner Wasserstoff als Hydriergas verwendet.

Das erfindungsgemäße Verfahren kann in Ab- oder Anwesenheit eines Lösungs- oder Verdünnungsmittels durchgeführt werden, d. h. es ist nicht erforderlich, das Verfahren in Lösung durchzuführen.

Vorzugsweise wird jedoch ein Lösungs- oder Verdünnungsmittel eingesetzt. Als Lösungs- oder Verdünnungsmittel kann jedes geeignete Lösungsmittel- oder Verdünnungsmittel eingesetzt werden. Die Auswahl ist dabei nicht kritisch, solange das eingesetzte Lösungs- oder Verdünnungsmittel in der Lage ist, mit der wenigstens einen Benzolpolycarbonsäure oder einem Derivat davon eine homogene Lösung zu bilden.

Beispielsweise können die Lösungs- oder Verdünnungsmittel auch Wasser enthalten. Beispielsweise ist ein

Lösungs- oder Verdünnungsmittel ausgewählt aus der Gruppe bestehend aus geradkettigen oder cyclischen Ethern, wie beispielsweise Tetrahydrofuran oder Dioxan, aliphatischen Alkoholen, in denen der Alkylrest vorzugsweise 1 bis 10 Kohlenstoffatome, insbesondere 3 bis 6 Kohlenstoffatome aufweist, beispielsweise i-Propanol, n-Butanol, i-Butanol, n-Hexanol und Mischungen davon.

Die Menge des eingesetzten Lösungs- oder Verdünnungsmittels ist nicht in besonderer Weise beschränkt und kann je nach Bedarf frei gewählt werden, wobei jedoch solche Mengen bevorzugt sind, die zu einer 10 bis 70 Gew.-%igen Lösung der wenigstens einen zur Hydrierung vorgesehenen Benzolpolycarbonsäure oder eines Derivates davon führen.

Besonders bevorzugt wird im Rahmen des erfindungsgemäßen Verfahrens das bei der Hydrierung gebildete Produkt, also die entsprechende Cyclohexanpolycarbonsäure oder ein Derivat davon als Lösungsmittel eingesetzt, gegebenenfalls neben anderen Lösungs- oder Verdünnungsmitteln. In jedem Fall kann ein Teil des im Verfahren gebildeten Produkts der noch zu hydrierenden Benzolpolycarbonsäure oder des Derivats davon beigemischt werden. Bezogen auf das Gewicht der zur Hydrierung vorgesehenen Verbindung wird vorzugsweise die 1- bis 30-fache, besonders bevorzugt die 5- bis 20-fache, insbesondere die 5- bis 10-fache Menge, des Umsetzungsproduktes als Lösungs- oder Verdünnungsmittel zugemischt.

Bei der erfindungsgemäß erhaltenen wenigstens einen Cyclohexanpolycarbonsäure oder einem Derivat davon handelt es sich bevorzugt um Verbindungen der Formel (I),

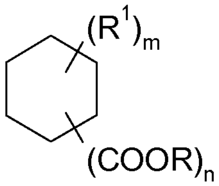

(I)

worin

| | |
|---|---|
| $R^1$ | $C_1$-$C_4$-Alkyl, |
| m | 0, 1, 2 oder 3, |
| n | 1, 2, 3 oder 4, und |
| R | Wasserstoff, $C_4$-$C_{12}$-Alkyl |

bedeuten.

Um diese Verbindungen der allgemeinen Formel (I) zu erhalten, werden erfindungsgemäß als Edukte die entsprechenden aromatischen Verbindungen der allgemeinen Formel (II) eingesetzt:

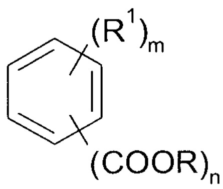

(II)

worin

| | |
|---|---|
| $R^1$ | $C_1$-$C_4$-Alkyl, |
| m | 0, 1, 2 oder 3, |
| n | 1, 2, 3 oder 4, und |
| R | Wasserstoff, $C_4$-$C_{12}$-Alkyl |

bedeuten.

Die Bedeutungen von $R^1$, R, m und n in den allgemeinen Formeln (I) und (II) werden im Folgenden gemeinsam diskutiert, wobei dem Fachmann bewusst ist, dass sich die Verbindungen der Formeln (I) und (II) durch die Anzahl der Ring-Wasserstoff-Atome unterscheiden.

Wenn m 2 oder 3 ist, können die Reste $R^1$ gleich oder verschieden sein. Die $C_1$-$C_4$-Alkylgruppen können geradkettig oder verzweigt sein. Wenn $R^1$ für eine Alkylgruppe steht, handelt es sich bevorzugt um Methyl-, Ethyl-, n-Propyl-, i-Propyl, n-Butyl, i-Butyl, sek.-Butyl oder tert.-Butyl. Bevorzugt ist m 0, d. h. es liegen keine $C_1$-$C_4$-Alkyl-Substituenten vor, sondern ausschließlich Wasserstoffatome, so dass ein aromatischer Benzolring (allgemeine Formel II) bzw. ein gesättigter Cyclohexylring (allgemeine Formel (I)) vorliegt.

Die n Reste R können gleich oder verschieden sein. Für den Fall, dass R Wasserstoff bedeutet, liegt eine Cyclohexanpolycarbonsäure bzw. Benzolpolycarbonsäure vor. Die $C_4$-$C_{12}$-Alkylgruppen können geradkettig oder verzweigt sein. R ist bevorzugt ein $C_6$-$C_{12}$-Alkyl, ganz besonders bevorzugt $C_8$-$C_{10}$-Alkyl. Beispiele für derartige Alkylgruppen sind n-Butyl, i-Butyl, sek.-Butyl oder tert.-Butyl, n-Pentyl, n-Hexyl, n-Octyl, 2-Ethyl-hexyl, n-Nonyl, iso-Nonyl, n-Decyl, iso-Decyl, n-Undecyl, iso-Undecyl, n-Dodecyl, iso-Dodecyl, n-Tridecyl, iso-Tridecyl, Stearyl, und n-Eicosyl.

Werden erfindungsgemäß Anhydride der wenigstens einen Benzolpolycarbonsäure oder eines Derivates davon eingesetzt, so sind zwei Carbonsäuregruppen unter Abspaltung eines Moleküls $H_2O$ miteinander verbunden. Dabei können die beiden Carbonsäuregruppen in einem Molekül (intramolekular) oder zwei Molekülen (Intermolekular) vorliegen. Dies gilt für die Edukte gemäß der allgemeinen Formel (II) und für die Produkte gemäß der allgemeinen Formel (I) und ist dem Fachmann bekannt.

Bei den Alkylgruppen kann es sich jeweils um einzelne Isomere der genannten Alkylgruppen oder um Gemische verschiedener Alkylgruppen handeln. Die verschiedenen Alkylgruppen können verschiedene Isomere mit derselben Zahl von Kohlenstoffatomen sein und/oder Alkylgruppen, die eine verschiedene Anzahl von Kohlenstoffatomen aufweisen.

Bei den erfindungsgemäß erhaltenen Cyclohexanpolycarbonsäuren oder Derivaten davon der allgemeinen Formel (I) handelt es sich um Mono-, Di-, Tri-, Tetraester und Anyhdride der Cyclohexanpolycarbonsäuren. Bevorzugt sind alle Carbonsäuregruppen verestert. Die eingesetzten Ester sind Alkylester, wobei bevorzugte Alkylgruppen R bereits vorstehend genannt sind.

Bevorzugt werden erfindungsgemäß Cyclohexanpolycarbonsäure-Derivate ausgewählt aus der Gruppe bestehend aus kernhydrierten Mono- und Dialkylestern der Phthalsäure, Isophthalsäure und Terephthalsäure, kernhydrierten Mono-, Di- und Trialkylestem der Trimellitsäure, der Trimesinsäure und der Hemimellitsäure oder Mono-, Di-, Tri- und Tetraalkylestern der Pyrromellitsäure erhalten, wobei die Alkylgruppen R die oben genannten Bedeutungen haben.

Die erfindungsgemäß bevorzugt eingesetzten Benzolpolycarbonsäuren sind insbesondere ausgewählt aus der Gruppe bestehend aus Phthalsäure, Isophthalsäure, Terephthalsäure, Trimellitsäure, Trimesinsäure, Hemimellitsäure, Pyromeliltsäure und Mischungen davon. Ganz besonders bevorzugt wird Phthalsäure eingesetzt. Die vorstehend genannten Säuren sind kommerziell erhältlich.

Weiter bevorzugt werden erfindungsgemäß Benzolpolycarbonsäureester der allgemeinen Formel (II) eingesetzt. Diese werden beispielsweise erhalten, indem wenigstens eine Benzolpolycarbonsäure ausgewählt aus der Gruppe bestehend aus Phthalsäure, Isophthalsäure, Terephthalsäure, Trimellitsäure, Trimesinsäure, Hemimellitsäure, Pyromeliltsäure und Mischungen davon, mit entsprechenden Alkoholen R-OH umgesetzt werden.

Als Alkohole werden bevorzugt die den Resten R der Cyclohexanpolycarbonsäure-Derivate der Formel I entsprechenden Alkohole eingesetzt.

Bevorzugt werden somit lineare oder verzweigte Alkohole mit $C_4$-$C_{12}$-Alkylresten eingesetzt. Bei den zur Veresterung mit den Benzolpolycarbonsäuren eingesetzten Alkoholen kann es sich jeweils um die den vorstehend genannten Resten R entsprechenden einzelnen Isomere der Alkohole oder um Gemische verschiedener Alkohole mit isomeren Alkylresten mit derselben Zahl von Kohlenstoffatomen handeln und/oder um Gemische verschiedener Alkohole mit unterschiedlicher Zahl der Kohlenstoffatome.

Die zur Umsetzung mit den Benzolpolycarbonsäuren geeigneten Alkohole oder Alkoholgemische können nach allen dem Fachmann bekannten Verfahren hergestellt werden. Geeignete Verfahren zur Herstellung von Alkoholen oder Verfahrensschritte, die bei der Herstellung von Alkoholen angewendet werden, sind zum Beispiel:

Hydroformylierung mit anschließender Hydrierung der gebildeten Aldehyde, zum Beispiel wie in WO 92/13818 offenbart;

Hydrierung von Aldolprodukten, zum Beispiel wie in DE-A 102 51 311 offenbart;

Hydratisierung von Alkenen, zum Beispiel wie in US 5,136,108 offenbart.

Hydrierung von Carbonsäuren und Carbonsäureestern, insbesondere Fettsäuren und Fettsäureestern, zum Beispiel wie in US 5,463,143 offenbart.

Hydrierung von ungesättigten Alkoholen oder von Carbonylverbindungen, zum Beispiel wie in EP-A 0 394 842 offenbart;

Hydrierung von Epoxiden, zum Beispiel wie in GB-A 879 803 offenbart;

Verfahren umfassend einen Telomerisationsschritt, zum Beispiel wie in US 3,091,628 offenbart;

Verfahren umfassend einen Isomerisierungsschritt, zum Beispiel wie in DE-A 42 28 887 offenbart;

Hydrolyse von Sulfaten, zum Beispiel wie in GB-A 1,165,309 offenbart;

Umsetzung von Dienen mit Aminen, zum Beispiel wie in DE-A 44 31 528 offenbart;

Enzymatische Herstellung von Alkoholen, zum Beispiel wie in WO 93/24644 offenbart;

Selektive Hydrierung von Dienen, zum Beispiel wie in US 3,203,998 offenbart;

Herstellung von Alkoholen aus Nitrilen, zum Beispiel wie in EP-A 0 271 092 offenbart;

Herstellung von Alkoholen durch Umsetzung von Alkinen, zum Beispiel wie in RU 205 9597-C1 offenbart; und

Hydrogenolyse von substituierten Tetrahydropyranen, zum Beispiel wie in GB 1,320,188 offenbart.

Dem Fachmann sind weitere Verfahren zur Herstellung von Alkoholen bekannt, die ebenfalls zur Veresterung mit Benzolpolycarbonsäuren geeigneten Alkoholen oder Alkoholgemischen eingesetzt werden können.

Bevorzugt eingesetzte Alkohole sind - wie vorstehend erwähnt - Alkohole, die $C_4$-$C_{12}$-Alkylreste aufweisen. Insbesondere die längerkettigen $C_5$-$C_{12}$-Alkohole bzw. Alkoholgemische, die diese Alkohole enthalten, werden besonders bevorzugt durch katalytische Hydroformylierung (auch als Oxoreaktion bezeichnet) von Olefinen und anschließende Hydrierung der gebildeten Aldehyde hergestellt.

Geeignete Hydroformylierungsverfahren sind dem Fachmann bekannt und sind in den vorstehend genannten Dokumenten offenbart. Die in den genannten Dokumenten offenbarten Alkohole und Alkoholgemische können mit den vorstehend genannten Benzolpolycarbonsäuren zu den gewünschten Benzolpolycarbonsäurealkylestern bzw. -estergemischen umgesetzt werden.

$C_5$-Alkohole bzw. Gemische, die $C_5$-Alkohole, besonders bevorzugt n-Pentanol enthalten, können zum Beispiel durch Hydroformylierung von Butadien in Anwesenheit einer wässrigen Lösung einer Rhodiumverbindung und eines Phosphins als Katalysator hergestellt werden. Ein solches Verfahren ist zum Beispiel in EP-A 0 643 031 offenbart.

Geeignete $C_7$-Alkoholmischungen, die zur Veresterung mit den Benzolpolycarbonsäuren eingesetzt werden können, sind zum Beispiel in JP-A 2000/319 444 offenbart. Die Herstellung der $C_7$-Alkoholmischung erfolgt durch Hydroformylierung mit anschließender Hydrierung der gebildeten Aldehyde.

Mischungen enthaltend $C_8$-Alkohole und deren Herstellungsverfahren sind zum Beispiel in GB-A 721 540 offenbart, worin ein Verfahren zur Herstellung von Isooctylalkoholen ausgehend von Heptenen mittels Hydroformylierung und anschließender Hydrierung beschrieben wird. Ein beispielhaft genanntes, weiteres Dokument, das die Herstellung von $C_7$-Alkoholen bzw. diese Alkohole enthaltenden Mischungen offenbart, ist DE-A 195 30 414.

Cg-Alkohole bzw. Mischungen enthaltend $C_9$-Alkohole werden bevorzugt durch Dimerisierung von Butenen, Hydroformylierung der erhaltenen Octene und anschließende Hydrierung des erhaltenen $C_9$-Aldehyds hergestellt.

Geeignete Verfahren und $C_9$-Alkohle enthaltende Mischungen sind zum Beispiel in WO 92/13818 offenbart.

$C_{10}$-Alkohole und Mischungen enthaltend diese Alkohole sind zum Beispiel in WO 2003/66642 offenbart.

$C_{12}$-Alkohole bzw. Mischungen enthaltend $C_{12}$-Alkohole, insbesondere Trimethylnonanol, und ein Verfahren zu dessen Herstellung sind zum Beispiel in WO 98/03462 offenbart.

Besonders bevorzugt werden erfindungsgemäß Dialkylester der vorstehend genannten Cyclohexandicarbonsäuren, insbesondere 1,2-, 1,3- oder 1,4-Dialkylester und ganz besonders bevorzugt 1,2-Dialkylester erhalten. Dabei können Dialkylester erhalten werden bzw. die entsprechenden Benzoldicarbonsäuredialkylester eingesetzt werden, worin beide Estergruppen der Dialkylester dieselben Alkylreste tragen, sowie Estergruppen, worin die beiden Estergruppen der Dialkylester unterschiedliche Alkylgruppen tragen. Beispiele für gemischte und nicht gemischte Dialkylester sind bereits vorstehend genannt. Weiterhin ist es möglich, dass die Alkylgruppen zwar die gleiche Kohlenstoffatomanzahl aufweisen, jedoch geradkettig sind oder unterschiedliche Verzweigungen aufweisen und somit Isomerengemische bilden. Solche Isomerengemische können auch eingesetzt werden, wenn die Kohlenstoffanzahl der Alkylgruppen der Dialkylester unterschiedlich ist. Der Anteil der verschiedenen Isomeren der Alkylgruppen ergibt sich im Allgemeinen aus der Zusammensetzung der Alkohole, die zur Veresterung der Benzoldicarbonsäuren eingesetzt werden, die nach Veresterung erfindungsgemäß zu den Cyclohexandicarbonsäureestern hydriert werden. Geeignete Alkoholmischungen sind vorstehend bereits genannt. Im Sinne der vorliegenden Anmeldung sind somit unter geradkettigen oder verzweigten Alkylresten mit einer bestimmten Anzahl von Kohlenstoffatomen neben den jeweils einzelnen Isomeren Isomerengemische zu verstehen, deren Zusammensetzung sich - wie vorstehend erwähnt - aus der Zusammensetzung der zur Veresterung der Benzoldicarbonsäuren eingesetzten Alkohole ergibt.

Unter geradkettigen Alkylresten sind im Sinne der vorliegenden Anmeldung ausschließlich geradkettige Alkylreste zu verstehen, jedoch auch Mischungen von Alkylresten, die überwiegend geradkettig sind.

Handelt es sich bei den Alkylresten R der Cyclohexanpolycarbonsäureester um $C_4$-Alkylreste, so werden diese durch Umsetzung der Benzolpolycarbonsäuren der Formel (II) mit R gleich Wasserstoff mit n-Butanol, iso-Butanol, sek.-Butanol oder tert.-Butanol erhalten. Dabei können zur Herstellung von Benzolpolycarbonsäureestern, worin R ein $C_4$ ist, jeweils Gemische der genannten Butanole eingesetzt werden oder einzelne Isomere. Bevorzugt werden einzelne Isomere des Butanols eingesetzt. Die Herstellung der vorstehend genannten $C_4$-Alkohole ist dem Fachmann bekannt.

Handelt es sich bei den Alkylresten R der Cyclohexanpolycarbonsäureester um $C_5$- bis $C_{12}$-Alkylreste, werden bevorzugt $C_5$- bis $C_{12}$-Alkohole eingesetzt, die Verzweigungsgrade (ISO-Index) von im Allgemeinen 0,10 bis 4, bevorzugt 0,5 bis 3, besonders bevorzugt 0,8 bis 2 und insbesondere bei 1 bis 1,5, aufweisen, d. h. im Allgemeinen handelt es sich bei den jeweiligen Alkoholen um Gemische verschiedener Isomere.

Ganz besonders bevorzugt werden $C_9$-Alkoholgemische mit einem ISO-Index vom 1 bis 2,5, insbesondere Nonanolgemische mit einem ISO-Index von 1,25 bzw. 1,6 eingesetzt. Der ISO-Index ist eine dimensionslose Größe, die mittels Gaschromatographie bestimmt wurde.

| | |
|---|---|
| Methode: | Kapillar GC |
| Apparatur: | Kapillar Gaschromatograph mit Autosampler, Split/Splitless-Injektionssystem und Flammenionisationsdetektor (FID) |
| Chemikalien: | MSTFA (N-Methyl-N-trimethylsilyltrifluoracetamid) entsprechende Vergleichssubstanzen zur Bestimmung der Retentionszeiten |
| Probenvorbereitung: | 3 Tropfen der Probe werden in 1 ml MSTFA und für 60 Minuten bei 80 °C gehalten |
| GC Bedingungen: | Kapillarsäule Ultra-1, Länge 50 m, Innendurchmesser 0,25 mm, Filmdicke 0,1 Mikrometer, Trägergas Helium, |
| Säulenvordruck | 200 psi constant, |
| Split | 80 ml/min, |
| Septumspülung | 3 ml/min, |
| Ofentemperatur | 120 °C, 25 min, isotherm, |
| Injektortemperatur | 250 °C, |
| Detektortemperatur | 250 °C (FID), |
| Injektionsvolumen | 0,5 Mikroliter |
| Berechnung: | Die Vorgehensweise bei der Berechnung des Iso-Index wird in der folgenden Tabelle ersichtlich |

Tabelle mit beispielhafter Berechnung des Iso-Index:

| Komponente | Name | Verzweigung | Anteil in Flächen-% | Index |
|---|---|---|---|---|
| **1** | 2-Ethyl-2-methylhexanol-1 | 2 | 1,00 | 0,0200 |
| **2** | 2-Ethyl-4-methylhexanol-1 | 2 | 1,00 | 0,0200 |
| **3** | 2-Ethyl-4-methylhexanol-1 | 2 | 1,00 | 0,0200 |
| **4** | 2-Propyl-3-methylpentanol-1 | 2 | 1,00 | 0,0200 |
| **5** | 2-Propyl-hexanol-1 | 1 | 1,00 | 0,0100 |
| **6** | 2,5-Dimethylheptanol-1 | 2 | 1,00 | 0,0200 |
| **7** | 2,3-Dimethylheptanol-1 | 2 | 1,00 | 0,0200 |
| **8** | 2,3,4-Trimethylhexanol-1 | 3 | 1,00 | 0,0300 |
| **9** | 2-Ethylheptanol-1 | 1 | 1,00 | 0,0100 |
| **10** | 3-Ethyl-4-methylhexanol-1 | 2 | 82,00 | 1,6400 |
| **11** | 3-Ethylheptanol-1 | 1 | 1,00 | 0,0100 |
| **12** | 2-Methyloctanol-1 | 1 | 1,00 | 0,0100 |
| **13** | 4,5-Dimethylheptanol-1 | 2 | 1,00 | 0,0200 |
| **14** | 4,5-Dimethylheptanol-1 | 2 | 1,00 | 0,0200 |

(fortgesetzt)

| Komponente | Name | Verzweigung | Anteil in Flächen-% | Index |
|---|---|---|---|---|
| **15** | 4-Methyloctanol-1 | 1 | 1,00 | 0,0100 |
| **15a** | 7-Methyloctanol-1 | 1 | 1,00 | 0,0000 |
| **16** | 6-Methyloctanol-1 | 1 | 1,00 | 0,0100 |
| **17** | Nonanol-1 | 0 | 1,00 | 0,0000 |
| | Unbekannte Komponente | 2 | 1,00 | 0,0200 |
| | Summe | | 99,00 | 1,9000 |
| | | | Iso-Index: | 1,9200 |

Die $C_5$- bis $C_{12}$-Alkohole werden gemäß den vorstehend genannten Verfahren hergestellt. Zur Herstellung von Benzolpolycarbonsäureestern, worin R ein $C_9$-Alkyrest ist, wird besonders bevorzugt ein Nonanol-Gemisch eingesetzt, worin 0 bis 20 Gew.-%, bevorzugt 0,5 bis 18 Gew.-%, besonders bevorzugt 6 bis 16 Gew.-% des Nonanol-Gemisches keine Verzweigung aufweisen, 5 bis 90 Gew.-%, bevorzugt 10 bis 80 Gew.-%, besonders bevorzugt 45 bis 75 Gew.-%, eine Verzweigung, 5 bis 70 Gew.-%, bevorzugt 10 bis 60 Gew.-%, besonders bevorzugt 15 bis 35 Gew.-% zwei Verzweigungen, 0 bis 10 Gew.-%, bevorzugt 0 bis 8 Gew.-%, besonders bevorzugt 0 bis 4 Gew.-% drei Verzweigungen aufweisen und 0 bis 40 Gew.-%, bevorzugt 0,1 bis 30 Gew.-%, besonders bevorzugt 0,5 bis 6,5 Gew.-%. sonstige Komponenten sind. Unter sonstigen Komponenten sind im Allgemeinen Nonanole mit mehr als drei Verzweigungen, Decanole oder Octanole zu verstehen, wobei die Summe der genannten Komponenten 100 Gew.-% ergibt.

Die vorliegende Erfindung betrifft daher das erfindungsgemäße Verfahren, wobei es sich bei dem wenigstens einen Derivat der Benzolpolycarbonsäure um Mono-, Di-, Tri-, Tetraester und Anyhdride der Benzolpolycarbonsäure handelt. Bevorzugt sind alle Carbonsäuregruppen verestert. Die eingesetzten Ester sind Alkylester, wobei bevorzugte Alkylgruppen R bereits vorstehend genannt sind.

Weiter bevorzugt betrifft die vorliegende Erfindung das erfindungsgemäße Verfahren, wobei es sich bei dem wenigstens einen Derivat einer Benzolpolycarbonsäure um Mono-, Di-, Tri-, Tetraester der Benzolpolycarbonsäure, wobei diese durch Umsetzung mit einem Nonanol-Gemisch, worin 0 bis 20 Gew.-%, bevorzugt 0,5 bis 18 Gew.-%, besonders bevorzugt 6 bis 16 Gew.-% des Nonanol-Gemisches keine Verzweigung aufweisen, 5 bis 90 Gew.-%, bevorzugt 10 bis 80 Gew.-%, besonders bevorzugt 45 bis 75 Gew.-%, eine Verzweigung, 5 bis 70 Gew.-%, bevorzugt 10 bis 60 Gew.-%, besonders bevorzugt 15 bis 35 Gew.-% zwei Verzweigungen, 0 bis 10 Gew.-%, bevorzugt 0 bis 8 Gew.-%, besonders bevorzugt 0 bis 4 Gew.-% drei Verzweigungen aufweisen und 0 bis 40 Gew.-%, bevorzugt 0,1 bis 30 Gew.-%, besonders bevorzugt 0,5 bis 6,5 Gew.-%. sonstige Komponenten sind, wobei die Summe der genannten Komponenten 100 Gew.-% ergibt, umgesetzt worden ist, handelt.

Eine besonders bevorzugte Ausführungsform eines Nonanol-Gemisches, das zur Herstellung von bevorzugt verwendeten Benzolpolycarbonsäurederivaten eingesetzt wird, weist die folgende Zusammensetzung auf:

1.73 bis 3,73 Gew.-%, bevorzugt 1,93 bis 3,53 Gew.-%, besonders bevorzugt 2,23 bis 3,23 Gew.-% 3-Ethyl-6-methyl-hexanol;

0,38 bis 1,38 Gew.-%, bevorzugt 0,48 bis 1,28 Gew.-%, besonders bevorzugt 0,58 bis 1,18 Gew.-% 2,6 Dimethylheptanol;

2,78 bis 4,78 Gew.-%, bevorzugt 2,98 bis 4,58 Gew.-%, besonders bevorzugt 3,28 bis 4,28 Gew.-% 3,5-Dimethylheptanol;

6,30 bis 16,30 Gew.-%, bevorzugt 7,30 bis 15,30 Gew.-%, besonders bevorzugt 8,30 bis 14,30 Gew.-% 3,6-Dimethylheptanol;

5.74 bis 11,74 Gew.-%, bevorzugt 6,24 bis 11,24 Gew.-%, besonders bevorzugt 6,74 bis 10,74 Gew.-% 4,6-Dimethylheptanol;

1,64 bis 3,64 Gew.-%, bevorzugt 1,84 bis 3,44 Gew.-%, besonders bevorzugt 2,14 bis 3,14 Gew.-% 3,4,5-Trimethylhexanol;

1,47 bis 5,47 Gew.-%, bevorzugt 1,97 bis 4,97 Gew.-%, besonders bevorzugt 2,47 bis 4,47 Gew.-% 3,4,5-Trimethylhexanol, 3-Methyl-4-ethylhexanol und 3-Ethyl-4-methylhexanol;

4,00 bis 10,00 Gew.-%, bevorzugt 4,50 bis 9,50 Gew.-%, besonders bevorzugt 5,00 bis 9,00 Gew.-% 3,4-Dimethylheptanol;

0,99 bis 2,99 Gew.-%, bevorzugt 1,19 bis 2,79 Gew.-%, besonders bevorzugt 1,49 bis 2,49 Gew.-% 4-Ethyl-5-methylhexanol und 3-Ethylheptanol;

2,45 bis 8,45 Gew.-%, bevorzugt 2,95 bis 7,95 Gew.-%, besonders bevorzugt 3,45 bis 7,45 Gew.-% 4,5-Dimethylheptanol und 3-Methyloctanol;
1,21 bis 5,21 Gew.-%, bevorzugt 1,71 bis 4,71 Gew.-%, besonders bevorzugt 2,21 bis 4,21 Gew.-% 4,5-Dimethylheptanol;
1,55 bis 5,55 Gew.-%, bevorzugt 2,05 bis 5,05 Gew.-%, besonders bevorzugt 2,55 bis 4,55 Gew.-% 5,6-Dimethylheptanol;
1,63 bis 3,63 Gew.-%, bevorzugt 1,83 bis 3,43 Gew.-%, besonders bevorzugt 2,13 bis 3,13 Gew.-% 4-Methyloctanol;
0,98 bis 2,98 Gew.-%, bevorzugt 1,18 bis 2,78 Gew.-%, besonders bevorzugt 1,48 bis 2,48 Gew.-% 5-Methyloctaonol;
0,70 bis 2,70 Gew.-%, bevorzugt 0,90 bis 2,50 Gew.-%, besonders bevorzugt 1,20 bis 2,20 Gew.-% 3,6,6-Trimethylhexanol;
1,96 bis 3,96 Gew.-%, bevorzugt 2,16 bis 3,76 Gew.-%, besonders bevorzugt 2,46 bis 3,46 Gew.-% 7-Methyloctanol;
1,24 bis 3,24 Gew.-%, bevorzugt 1,44 bis 3,04 Gew.-%, besonders bevorzugt 1,74 bis 2,74 Gew.-% 6-Methyloctanol;
0,1 bis 3 Gew.-%, bevorzugt 0,2 bis 2 Gew.-%, besonders bevorzugt 0,3 bis 1 Gew.-% n-Nonanol;
25 bis 35 Gew.-%, bevorzugt 28 bis 33 Gew.-%, besonders bevorzugt 29 bis 32 Gew.-% sonstige Alkohole mit 9 und 10 Kohlenstoffatomen;

wobei die Gesamtsumme der genannten Komponenten 100 Gew.-% ergibt.

Ein solches Isononanol-Gemisch liegt verestert mit Phthalsäure im Diisononylphthalat der CAS Nr. 68515-48-0 vor, aus dem der Cyclohexan-1,2-dicarbonsäurediisononylester mit entsprechender Isononyl-Komponente durch das erfindungsgemäße Verfahren durch Hydrierung des aromatischen Kerns erzeugt werden kann. Solche Isononanol-Gemische können über den Weg der Zeolith-katalysierten Oligomerisierung von $C_2$-, $C_3$- und $C_4$-Olefingemischen, dem sogenannten Polygas-Prozess, Gewinnung einer $C_8$-Fraktion aus dem Oligomerisat und deren anschließende Hydroformylierung und Hydrierung erhalten werden.

Eine weitere besonders bevorzugte Ausführungsform eines Nonanol-Gemisches, das zur Herstellung von bevorzugt verwendeten Benzolpolycarbonsaure-Derivaten eingesetzt wird, weist die folgende Zusammensetzung auf:

6,0 bis 16,0 Gew.-%, bevorzugt 7,0 bis 15,0 Gew.-%, besonders bevorzugt 8,0 bis 14,0 Gew.-% n-Nonanol;
12,8 bis 28,8 Gew.-%, bevorzugt 14,8 bis 26,8 Gew.-%, besonders bevorzugt 15,8 bis 25,8 Gew.-% 6-Methyloctanol;
12,5 bis 28,8 Gew.-%, bevorzugt 14,5 bis 26,5 Gew.-%, besonders bevorzugt 15,5 bis 25,5 Gew.-% 4-Methyloctanol;
3,3 bis 7,3 Gew.-%, bevorzugt 3,8 bis 6,8 Gew.-%, besonders bevorzugt 4,3 bis 6,3 Gew.-% 2-Methyloctanol;
5,7 bis 11,7 Gew.-%, bevorzugt 6,3 bis 11,3 Gew.-%, besonders bevorzugt 6,7 bis 10,7 Gew.-% 3-Ethylheptanol;
1,9 bis 3,9 Gew.-%, bevorzugt 2,1 bis 3,7 Gew.-%, besonders bevorzugt 2,4 bis 3,4 Gew.-% 2-Ethylheptanol;
1.7 bis 3,7 Gew.-%, bevorzugt 1,9 bis 3,5 Gew.-%, besonders bevorzugt 2,2 bis 3,2 Gew.-% 2-Propylhexanol;
3,2 bis 9,2 Gew.-%, bevorzugt 3,7 bis 8,7 Gew.-%, besonders bevorzugt 4,2 bis 8,2 Gew.-% 3,5-Dimethylheptanol;
6,0 bis 16,0 Gew.-%, bevorzugt 7,0 bis 15,0 Gew.-%, besonders bevorzugt 8,0 bis 14,0 Gew.-% 2,5-Dimethylheptanol;
1.8 bis 3,8 Gew.-%, bevorzugt 2,0 bis 3,6 Gew.-%, besonders bevorzugt 2,3 bis 3,3 Gew.-% 2,3-Dimethylheptanol;
0,6 bis 2,6 Gew.-%, bevorzugt 0,8 bis 2,4 Gew.-%, besonders bevorzugt 1,1 bis 2,1 Gew.-% 3-Ethyl-4-methylhexanol;
2,0 bis 4,0 Gew.-%, bevorzugt 2,2 bis 3,8 Gew.-%, besonders bevorzugt 2,5 bis 3,5 Gew.-% 2-Ethyl-4-methylhexanol;
0,5 bis 6,5 Gew.-%, bevorzugt 1,5 bis 6 Gew.-%, besonders bevorzugt 1,5 bis 5,5 Gew.-% sonstige Alkohole mit 9 Kohlenstoffatomen;

wobei die Gesamtsumme der genannten Komponenten 100 Gew.-% ergibt.

Ein solches Isononanol-Gemisch liegt verestert mit Phthalsäure im Düsononylphthalat der CAS Nr. 28553-12-0 vor, aus dem der Cyclohexan-1,2-dicarbonsäurediisononylester mit entsprechender Isononyl-Komponente durch die erfindungsgemäße Hydrierung des aromatischen Kerns erzeugt werden kann. Solche Isononanol-Gemische können über den Weg der Dimerisierung von vorwiegend n-Butenen zu Octen-Gemischen mittels Nickel-haltigen Katalysatoren, beispielsweise nach dem Verfahren der WO 95/14647, anschließende Hydroformylierung des erhaltenen Octen-Gemisches, vorzugsweise Kobalt-katalysierte Hydroformylierung, und Hydrierung erhalten werden.

Ganz besonders bevorzugte Produkte des erfindungsgemäßen Verfahrens sind ausgewählt aus der Gruppe bestehend aus Cyclohexan-1,2-dicarbonsäuredi-n-octylester, Cyclohexan-1,2-dicarbonsäurediisooctylester, Cyclohexan-1,2-dicarbonsäuredi- (2-ethylhexyl)-ester, Cyclohexan-1,2-dicarbonsäuredi-n-nonylester, Cyclohexan-1,2-dicarbonsäurediisononylester, Cyclohexan-1,2-dicarbonsäuredi-(2-propylheptyl)-ester, Cyclohexan-1,2-dicarbonsäuredi-n-decyl-ester, Cyclohexan-1,2-dicarbonsäurediisodecylester und Mischungen davon.

Weitere erfindungsgemäß bevorzugt erhaltene Produkte sind die in der WO 99/32427 offenbarten, im Folgenden nochmals aufgelisteten Cyclohexan-1,2-dicarbonsäureester:

Cyclohexan-1,2-dicarbonsäuredi(isononyl)ester, erhältlich durch die erfindungsgemäße Hydrierung eines Di(isononyl)phthalats mit der CAS Nr. 68515-48-0;

Cyclohexan-1,2-dicarbonsäuredi(isononyl)ester, erhältlich durch die erfindungsgemäße Hydrierung eines Di(isononyl)phthalats mit der CAS Nr. 28553-12-0, basierend auf n-Buten;

Cyclohexan-1,2-dicarbonsäuredi(isononyl)ester, erhältlich durch die erfindungsgemäße Hydrierung eines Di(isononyl)phthalats mit der CAS Nr. 28553-12-0 basierend auf Isobuten;

ein 1 ,2-Di-$C_9$-Ester der Cyclohexandicarbonsäure, erhältlich durch die erfindungsgemäße Hydrierung eines Di(nonyl)phthalats mit der CAS Nr. 68515-46-8;

ein Cyclohexan-1,2-dicarbonsäuredi(isodecyl)ester erhältlich durch die erfindungsgemäße Hydrierung eines Di(isodecyl)phthalats mit der CAS Nr. 68515-49-1;

ein 1,2-Di(isodecyl)cyclohexandicarbonsäureester, erhältlich durch die erfindungsgemäße Hydrierung eines Di(isodecyl)phthalats, das hauptsächlich aus Di-(2-propylheptyl)phthalat besteht;

Des Weiteren sind auch die kommerziell erhältlichen Benzolcarbonsäureester mit den Handelsnamen Jayflex DINP (CAS Nr. 68515-25 48-0), Jayflex DIDP (CAS Nr. 68515-49-1), Palatinol 9-P, Vestinol 9 (CAS Nr. 28553-12-0), Palatinol N (CAS Nr. 28553-12-0), Jayflex DIOP (CAS Nr. 27554-26-3), Palatinol AH (CAS Nr. 117-81-7) und Palatinol Z (CAS Nr. 26761-40-0) geeignete Edukte für das erfindungsgemäße Verfahren.

Die erfindungsgemäß hergestellten Cyclohexanpolycarbonsäuren oder Derivate davon zeichnen sich gegenüber kommerziell verfügbaren Cyclohexanpolycarbonsäuren oder Derivaten davon durch einen geringeren Anteil an Nebenkomponenten aus, insbesondere an der Nebenkomponente Hexahydrophthalid und Isononylalkohol, und weisen dadurch bei der Verwendung als Weichmacher vorteilhaftere anwendungstechnische Eigenschaften, wie eine geringere Flüchtigkeit und eine bessere Verträglichkeit mit Kunststoffen, beispielweise PVC, auf.

Die erfindungsgemäß hergestellte wenigstens eine Cyclohexanpolycarbonsäure oder ein Derivat davon weist in einer bevorzugten Ausführungsform einen Gehalt an Hexahydrophthalid von höchstens 0,06 Fl.-% bevorzugt weniger als 0,04 FL-%, besonders bevorzugt 0,01 bis 0,03 Fl.-%, auf.

Die erfindungsgemäß hergestellte wenigstens eine Cyclohexanpolycarbonsäure oder ein Derivat davon weist in einer bevorzugten Ausführungsform einen Gehalt an Isonanol von höchstens 0,2 Fl-%, bevorzugt weniger als 0,15 Fl.-%, besonders bevorzugt 0,05 Fl.-% bis 0,13 Fl.-%, auf, falls erfindungsgemäß ein Cyclohexanpolycarbonsäureisononanolester hergestellt wird. Dadurch sind die erfindungsgemäß hergestellten Cyclohexanpolycarbonsäuren oder Derivate davon besonders gut für Anwendungen in Kontakt mit dem Menschen geeignet, z. B. für Kinderspielzeug, Lebensmittelverpackungen oder medizinische Artikel, insbesondere als Weichmacher in Kunststoffen.

Beispiele

Im Folgenden soll nunmehr das erfindungsgemäße Verfahren anhand einiger Ausführungsbeispiele näher erläutert werden. Die Versuche 4-6 wurden erfindungsgemäß durchgeführt, bei den Versuchen 1-3 handelt es sich um Vergleichsversuche, in allen Fällen ist eine Verringerung der unerwünschten Nebenprodukte (Isononanol und Hexahydrophthalid) um etwa 50% oder mehr zu beobachten. Entsprechend liegt der Gehalt des Wertproduktes (Diisononylcyclohexandicarboxylat) in den erfindungsgemäßen Versuchen 4-6 bei >99,5%.

Katalysator 1 wird gemäß WO2011/082991, Beispiel 1, hergestellt.

Katalysator 2 (Vergleich) wird gemäß DE19624485, Herstellungsbeispiel, hergestellt.

Hydrierbeispiele

Die Hydrierungen werden in einer Kaskade von 8 Rohren (Innendurchmesser 6 mm, Länge 150 cm) durchgeführt. Dabei wurden die ersten sechs Rohre in Reihe als Hauptreaktor mit Umlauf betrieben, d.h. der Austrag des sechsten Rohrs wurde teilweise auf das erste Rohr zurückgeführt. Die letzten 2 Rohre wurden als Nachreaktor im geraden Durchgang betrieben. Jedes Rohr wurde mit 30 mL Katalysator befüllt.

Die Hydrierung wurde mit reinem Wasserstoff durchgeführt. Der Zulauf wurde so gewählt, dass die Katalysatorbelastung im Hauptreaktor (kg(Diisonylphtalat)/(L(Katalysator)·h) den in der nachfolgenden Tabelle angegeben Wert erreicht. Die Rückführrate wurde so gewählt, dass die Leerrohr-Geschwindigkeit im Hauptreaktor bei den in der Tabelle 1 angegebenen Werten liegt. Der Wasserstoff wurde druckgeregelt bei dem in der Tabelle 1 angegebenen Druck zugeführt. Die Reaktionstemperaturen sind ebenfalls in der Tabelle 1 angegeben.

**Tabelle 1: Hydrierversuche**

| Nr. | Kat. | Katalysatorbelastung [kg/(L·h)] | Leerrohr-Geschwindigkeit [m/h] | T im Hauptreaktor [°C] | T im Nachreaktor [°C] | Druck [bar] |
|---|---|---|---|---|---|---|
| 1 | 2 (Vgl.) | 0,5 | 55 | 111 | 125 | 240 |
| 2 | 1 | 0,73 | 55 | 126 | 155 | 240 |
| 3 | 1 | 0,5 | 55 | 111 | 125 | 240 |
| 4 | 1 | 0,5 | 34 | 111 | 125 | 36 |
| 5 | 1 | 0,5 | 34 | 120 | 140 | 36 |
| 6 | 1 | 0,25 | 34 | 111 | 125 | 36 |
| Katalysatorbelastung: kg (Diisononylphthalat)/(L(Katalysator) Stunde)<br>Leerrohr-Geschwindigkeit: $m^3$(Diisononylphthalat)/($m^2$(Reaktor-Querschnitt)/Zeit(h)) | | | | | | |

Tabelle 2: Ergebnisse der Hydrierversuche

| GC-Methode:<br>Säule: DB-1 30m, ID 0,32 mm, FD 0,25 μm<br>Temperaturprogramm: 50°C, 4 min - 5°C/min - 290°C - 28 min<br>Injektionsvolumen: 1 μL<br>Inlet-Temperatur: 300°C, Detektortemperatur (FID): 300°C<br>Isononanol: 13-18 min<br>Hexahydrophthalid: 20 min<br>Cyclohexan-1,2-dicarbonsäurediisononylester (DINCH): 47-54 min | | | | |
|---|---|---|---|---|
| Nr. | Isononanol [GC-Flächen-%] | Hexahydrophthalid [GC-Flächen-%] | DINCH-Gehalt [GC-Flächen-%] | Summe Sonstige [GC-Flächen-%] |
| 1 | 0,073 | 0,449 | 99,2 | 0,278 |
| 2 | 0,776 | 0,181 | 98,6 | 0,443 |
| 3 | 0,077 | 0,282 | 99,4 | 0,241 |
| 4 | 0,019 | 0,097 | 99,7 | 0,184 |
| 5 | 0,028 | 0,109 | 99,6 | 0,263 |
| 6 | 0,029 | 0,113 | 99,7 | 0,158 |

## Patentansprüche

**1.** Kontinuierliches Verfahren zur Herstellung von wenigstens einer Cyclohexanpolycarbonsäure oder einem Derivat davon durch Inkontaktbringen wenigstens einer entsprechenden Benzolpolycarbonsäure oder eines Derivates davon mit einem Wasserstoff enthaltenden Gas in Gegenwart wenigstens eines Schalenkatalysators, enthaltend ein Aktivmetall ausgewählt aus der Gruppe bestehend aus Ruthenium, Rhodium, Palladium, Platin und Mischungen davon, aufgebracht auf ein Trägermaterial enthaltend Siliziumdioxid, wobei das Porenvolumen des Trägermaterials 0,6 bis 1,0 ml/g, bestimmt durch Hg-Porosimetrie, beträgt, die BET-Oberfläche 280 bis 500 $m^2$/g beträgt, mindestens 90% der vorhandenen Poren einen Durchmesser von 6 bis 12 nm aufweisen, und 40 bis 70 Gew.-% des Aktivmetalls, bezogen auf die Gesamtmenge des Aktivmetalls, in der Schale des Katalysators bis zu einer Eindringtiefe von 200 μm vorliegen, **dadurch gekennzeichnet, dass** das Inkontaktbringen bei einer Leerrohrgeschwindigkeit von 20 bis 50 m/h erfolgt, wobei es sich bei dem wenigstens einen Derivat der Benzolpolycarbonsäure um Mono-, Di-, Tri-, Tetraester und Anyhdride der Benzolpolycarbonsäure handelt.

2. Verfahren nach Anspruch 1 , **dadurch gekennzeichnet, dass** das Verfahren bei einer Leerrohrgeschwindigkeit von 20 bis 45 m/h, ganz besonders bevorzugt von 20 bis 40 m/h, durchgeführt wird.

3. Verfahren nach Anspruch 1 oder 2, **dadurch gekennzeichnet, dass** das Inkontaktbringen bei einer Temperatur von 50 bis 250 °C, vorzugsweise 70 bis 180 °C und einem Druck oberhalb von 10 bar, vorzugsweise zwischen 20 bis 80 bar, besonders bevorzugt zwischen 30 und 50 bar, durchgeführt wird.

4. Verfahren nach einem der Ansprüche 1 bis 3, **dadurch gekennzeichnet, dass** es sich bei dem wenigstens einen Derivat einer Benzolpolycarbonsäure um Mono-, Di-, Tri-, Tetraester der Benzolpolycarbonsäure handelt, wobei diese durch Umsetzung mit einem Nonanol-Gemisch, worin 0 bis 20 Gew.-%, bevorzugt 0,5 bis 18 Gew.-%, besonders bevorzugt 6 bis 16 Gew.-% des Nonanol-Gemisches keine Verzweigung aufweisen, 5 bis 90 Gew.-%, bevorzugt 10 bis 80 Gew.-%, besonders bevorzugt 45 bis 75 Gew.-%, eine Verzweigung, 5 bis 70 Gew.-%, bevorzugt 10 bis 60 Gew.-%, besonders bevorzugt 15 bis 35 Gew.-% zwei Verzweigungen, 0 bis 10 Gew.-%, bevorzugt 0 bis 8 Gew.-%, besonders bevorzugt 0 bis 4 Gew.-% drei Verzweigungen aufweisen und 0 bis 40 Gew.-%, bevorzugt 0,1 bis 30 Gew.-%, besonders bevorzugt 0,5 bis 6,5 Gew.-%. sonstige Komponenten sind, wobei die Summe der genannten Komponenten 100 Gew.-% ergibt, erhalten werden.

## Claims

1. A continuous process for preparing at least one cyclohexanepolycarboxylic acid or a derivative thereof by bringing at least one corresponding benzenepolycarboxylic acid or a derivative thereof into contact with a hydrogen-comprising gas in the presence of at least one coated catalyst comprising an active metal selected from the group consisting of ruthenium, rhodium, palladium, platinum and mixes thereof applied to a support material comprising silicon dioxide, where the pore volume of the support material is from 0.6 to 1.0 ml/g, determined by Hg porosimetry, the BET surface area is from 280 to 500 $m^2$/g, at least 90% of the pores present have a diameter of from 6 to 12 nm, and from 40 to 70% by weight of the active metal, based on the total amount of the active metal, are present in the shell of the catalyst to a penetration depth of 200μm, wherein the contacting is carried out at a superficial velocity of from 20 to 50 m/h, where the at least one derivative of the benzenepolycarboxylic acid is a monoester, diester, triester, tetraester or anhydride of the benzenepolycarboxylic acid.

2. The process according to claim 1, wherein the process is carried out at a superficial velocity of from 20 to 45 m/h, very particularly preferably from 20 to 40 m/h.

3. The process according to claim 1 or 2, wherein the contacting is carried out at a temperature of from 50 to 250°C, preferably from 70 to 180°C, and a pressure above 10 bar, preferably in the range from 20 to 80 bar, particularly preferably from 30 to 50 bar.

4. The process according to any of claims 1 to 4, wherein the at least one derivative of a benzenepolycarboxylic acid is a monoester, diester, triester or tetraester of the benzenepolycarboxylic acid, which ester has been obtained by reaction with a nonanol mixture in which from 0 to 20% by weight, preferably from 0.5 to 18% by weight, particularly preferably from 6 to 16% by weight, of the nonanol mixture have no branching, from 5 to 90% by weight, preferably from 10 to 80% by weight, particularly preferably from 45 to 75% by weight, have one branching point, from 5 to 70% by weight, preferably from 10 to 60% by weight, particularly preferably from 15 to 35% by weight, have two branching points, from 0 to 10% by weight, preferably from 0 to 8% by weight, particularly preferably from 0 to 4% by weight, have three branching points and from 0 to 40% by weight, preferably from 0.1 to 30% by weight, particularly preferably from 0.5 to 6.5% by weight, are other components, with the sum of the components mentioned being 100% by weight.

## Revendications

1. Procédé continu de fabrication d'au moins un acide cyclohexanepolycarboxylique ou d'un dérivé de celui-ci par mise en contact d'au moins un acide benzènepolycarboxylique correspondant ou d'un dérivé de celui-ci avec un gaz contenant de l'hydrogène en présence d'au moins un catalyseur à enveloppe, contenant un métal actif choisi dans le groupe constitué par le ruthénium, le rhodium, le palladium, le platine et leurs mélanges, appliqué sur un matériau support contenant du dioxyde de silicium, le volume de pores du matériau support étant de 0,6 à 1,0 ml/g, déterminé par porosimétrie Hg, la surface BET étant de 280 à 500 $m^2$/g, au moins 90 % des pores présents présentant

un diamètre de 6 à 12 nm, et 40 à 70 % en poids du métal actif, par rapport à la quantité totale du métal actif, étant présent dans l'enveloppe du catalyseur jusqu'à une profondeur de pénétration de 200 μm, **caractérisé en ce que** la mise en contact a lieu à une vitesse en tube vide de 20 à 50 m/h, ledit au moins un dérivé de l'acide benzènepolycarboxylique correspondant à des mono-, di-, tri-, tétraesters et anhydrides de l'acide benzènepolycarboxylique.

**2.** Procédé selon la revendication 1, **caractérisé en ce que** le procédé est réalisé à une vitesse en tube vide de 20 à 45 m/h, de manière tout particulièrement préférée de 20 à 40 m/h.

**3.** Procédé selon la revendication 1 ou 2, **caractérisé en ce que** la mise en contact est réalisée à une température de 50 à 250 °C, de préférence de 70 à 180 °C, et à une pression supérieure à 10 bar, de préférence comprise entre 20 et 80 bar, de manière particulièrement préférée entre 30 et 50 bar.

**4.** Procédé selon l'une quelconque des revendications 1 à 3, **caractérisé en ce que** ledit au moins un dérivé d'un acide benzènepolycarboxylique consiste en des mono-, di-, tri-, tétraesters de l'acide benzènepolycarboxylique, ceux-ci étant obtenus par mise en réaction avec un mélange de nonanols, 0 à 20 % en poids, de préférence 0,5 à 18 % en poids, de manière particulièrement préférée 6 à 16 % en poids, du mélange de nonanols ne comprenant pas de ramification, 5 à 90 % en poids, de préférence 10 à 80 % en poids, de manière particulièrement préférée 45 à 75 % en poids une ramification, 5 à 70 % en poids, de préférence 10 à 60 % en poids, de manière particulièrement préférée 15 à 35 % en poids, deux ramifications, 0 à 10 % en poids, de préférence 0 à 8 % en poids, de manière particulièrement préférée 0 à 4 % en poids, trois ramifications, et 0 à 40 % en poids, de préférence 0,1 à 30 % en poids, de manière particulièrement préférée 0,5 à 6,5 % en poids étant d'autres composants, la somme des composants mentionnés étant de 100 % en poids.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 5286898 A
- US 5319129 A
- DE 2823165 A
- US 3027398 A
- EP 0603825 A
- EP 1042273 A
- DE 1263296 A
- US 20080188601 A1
- WO 2011082991 A2
- WO 2011082991 A
- WO 9213818 A
- DE 10251311 A
- US 5136108 A
- US 5463143 A
- EP 0394842 A
- GB 879803 A
- US 3091628 A
- DE 4228887 A
- GB 1165309 A
- DE 4431528 A
- WO 9324644 A
- US 3203998 A
- EP 0271092 A
- RU 2059597 C1
- GB 1320188 A
- EP 0643031 A
- JP 2000319444 A
- GB 721540 A
- DE 19530414 A
- WO 200366642 A
- WO 9803462 A
- WO 9514647 A
- WO 9932427 A
- DE 19624485


### In der Beschreibung aufgeführte Nicht-Patentliteratur


- **VON J.W. NIEMANTSVERDRIET.** Spectroscopy in Catalysis. VCH, 1995
- **VON S. AMELINCKX.** Handbook of Microscopy
- *CHEMICAL ABSTRACTS,* 68515-48-0
- *CHEMICAL ABSTRACTS,* 28553-12-0
- *CHEMICAL ABSTRACTS,* 68515-46-8
- *CHEMICAL ABSTRACTS,* 68515-49-1
- *CHEMICAL ABSTRACTS,* 68515-25 48-0
- *CHEMICAL ABSTRACTS,* 27554-26-3
- *CHEMICAL ABSTRACTS,* 117-81-7
- *CHEMICAL ABSTRACTS,* 26761-40-0